# EUROPÄISCHE PATENTANMELDUNG

(11) **EP 2 474 329 A1**
(43) Veröffentlichungstag der Anmeldung: **11.07.2012**
(21) Anmeldenummer: 12150259.5
(22) Anmeldetag: 05.01.2012
(51) Int. Cl.: A61L 2/03, C02F 1/461

(54) **Batterie betriebenes Handsprühgerät und seine Verwendung**

(30) Priorität: 06.01.2011 CH 302011
(71) Anmelder: pro aqua Diamantelektroden Produktion GmbH & Co KG, 8712 Niklasdorf (AT)
(72) Erfinder: Steffen, Hanspeter, 3473 Alchenstorf (CH); Schelch, Michael, 8600 Oberaich (AT); Staber, Wolfgang, 8600 Bruck an der Mur (AT); Hermann, Robert, 8600 Oberaich (AT); Wesner, Wolfgang, 1050 Wien (AT); Ratz, Gerald, 9162 Strau (AT)
(74) Vertreter: Vinazzer, Edith

(57) **Zusammenfassung**

Batterie betriebenes Handsprühgerät mit einem Flüssigkeitsbehälter (A), einem Sprühkopf (B) mit Sprühdüse (B₁), einer Elektrolysezelle (C₁), einer Wasserpumpe (C₂), Steigrohr und Leitungen, einer elektrischen/elektronischen Steuerung (G) mit einem Fingerabzug (F) und einem Batteriefach (E). Die Elektrolysezelle (C₁) enthält dotierte Volldiamant-Elektroden.

## Beschreibung

Die Erfindung betrifft ein Batterie betriebenes Handsprühgerät mit einem Flüssigkeitsbehälter, einem Sprühkopf mit Sprühdüse, einer Wasserpumpe, einer Elektrolysezelle, Steigrohr und Leitungen, einer elektrischen/elektronischen Steuerung, mit einem Fingerabzug und einem Batteriefach, für die Herstellung von neutralem Elektrolysewasser zur Reinigung und Desinfektion von Oberflächen.

Bislang konnten Oberflächen, Materialien und Gegenstände mittels eines Handsprühgerätes mit chemischen Produkten und Desinfektionsmitteln oder mit Heisswasser hygienisch gereinigt werden, was sehr kostspielig ist und zudem eine enorme Belastung für die Umwelt durch Chemikalien enthaltende Abwässer darstellt, die schwierig zu reinigen sind und biologische Reinigungsstufen in der Abwasserreinigung belasten. Zudem sind solche chemischen Produkte zur Reinigung und Desinfektion oftmals toxisch und verursachen bei Anwendern im Dauereinsatz Allergien und Ätzungen und Ekzeme. Auch sind die Kosten und der Aufwand an Verpackungsmaterial für diese Chemie-Produkte sehr hoch und belasten die Umwelt beträchtlich.

Es sind ferner Batterie betriebene Handsprühgeräte bekannt, beispielsweise ist aus der WO 2009/155 545 A2 ein Handsprühgerät der eingangs genannten Art bekannt. Dieses Handsprühgerät weist einen Flüssigkeitsbehälter für Wasser, einen Sprühkopf mit einer Sprühdüse, eine im Behälter positionierte Elektrolysezelle, eine elektrische Wasserpumpe, Schläuche als Saugrohr und Verbindungsleitungen, eine Batterie mit entsprechenden elektrischen Verbindungen, einen Fingerabzug als Elektroschalter und eine elektrische Steuerung auf. Ein zwischen der Batterie und der Elektrolysezelle eingebauter DC-to-DC-Converter stellt eine höhere Spannung zur Verfügung, die größer ist als die von der Batterie gelieferte Spannung. Die Elektroden in der Elektrolysezelle bestehen aus einem leitfähigen Polymer, aus Titan oder aus platinbeschichtetem Titan. Mit einem derartigen Handsprühgerät lassen sich keine effektive Reinigungswirkung und keine Desinfektion erzielen.

Der Erfindung liegt die Aufgabe zu Grunde, ein Handsprühgerät zur Verfügung zu stellen, mit welchem Oberflächen, Materialien und Gegenstände effektiv gereinigt, hygienisiert, und desinfiziert werden können, ohne den teuren Einsatz von Umwelt belastenden und toxischen Chemikalien und Energie verschwendendem Heisswasser.

Gelöst wird die gestellte Aufgabe erfindungsgemäß dadurch, dass die Elektrolysezelle dotierte Volldiamant-Elektroden enthält.

Dank dem Einsatz von Volldiamant-Elektroden ist es mit der Erfindung möglich, direkt im Handsprühgerät eine starke Biozid-Lösung herzustellen mit einem mikrobiellen ReduktionsPotential von 6 bis 10 log. Dank der starken Biozid -Wirkung des Elektrolyse-Wassers, das durch die Diamant-Elektroden direkt in der Handsprühflasche hergestellt wird, können wegen der erhöhten Lösungskraft des Wassers nicht nur Schmutz auf Oberflächen leicht entfernt werden (Reduktion der Wasserstoffketten von 14 Moleküle auf 3 Moleküle per Cluster ergibt eine 4- fache Steigerung der molekularen Dipolwirkungen), sondern auch Pilze, Bakterien, Viren und deren Sporen vollständig durch Kaltoxidation vernichtet werden.

Nur mit Wasser und elektrischer Energie und gegebenenfalls mit Zusatz von etwas Salz kann mit dem Handsprühgerät eine Reinigung und gleichzeitig eine Desinfektion von Oberflächen und anderen Objekten, ohne den Einsatz von Chemikalien und ohne Rückstände zu bilden, erreicht werden.

Es gibt nur wenige Anoden-Elektroden, die während der Elektrolyse inert bleiben - also nicht in Lösung gehen. Bor dotierter Voll- Diamant ist ein Material, das sich während einer Elektrolyse weitgehend nicht auflöst.

Diese Reinigungs- und Desinfektions- Technik mit dieser neuen Entwicklung des erfindungsgemäßen Handsprühgerätes ist sehr ökologisch und hat keinen negativen "Footprint" bezüglich der CO₂ Bilanz und ist deshalb äusserst nachhaltig.

Elektrolytisch oxidatives Wasser (EOW) oder chemisch aktives Wasser zerstört Mikroorganismen, wie Viren, Bakterien, Pilze, Hefen und Einzeller durch oxidative Radikale nicht chemisch, sondern physikalisch. Wegen seines hohen oxidativen Reduktionspotentials (ORP) beschädigt "aktives Wasser" die Zellwand-Membranen von Pathogenen. Der Krankheitserreger ist komprimitiert, was zu einer osmotischen oder hydrogenen Überlastung im Zellinneren führt. Die beschädigten Zellmembranen erlauben einen erhöhten Wassertransfer zwischen den Zellmembranen, was zu einer hydrogenen Überflutung der Zellen führt, und diese schneller gefüllt werden, als die Zellen sich des Wassers entledigen können. Diese Tatsache führt zu einem Zerplatzen der Zellen, respektive zum Zelltod durch Druckexplosion in wenigen Sekunden. Da es sich um ein physikalisches Zerstörungsprinzip handelt, ergeben sich nachweislich keine Resistenzen bei Pathogenen.

Die Erfindung zeigt eine neue Gerätetechnik auf zur Chemie- und Rückstand freien Reinigung, Hygienisierung, Desinfektion und Geruchsneutralisation von Oberflächen, Materialien und Gegenständen mittels eines Batterie betriebenen Handsprühgerätes unter Verwendung von elektrolysiertem kaltem oder warmem Wasser und mit Hilfe von oxidativen Radikalen, produziert in einer Elektrolysezelle mit Diamant-Elektroden, integriert im Sprühkopf des Handsprühgerätes. Die Erfindung zeigt ferner auf die spezifischen Eigenschaften des elektrolysierten, oxidativen Wassers, dessen Herstellung, gegebenenfalls dessen Salzkonzentration und Salzzusammensetzung, dessen Redoxpotential, respektive dessen Konzentration in freien oxidativen Radikalen und Gesamtkonzentration der oxidativen Radikale, und dessen pH-Wert und Aufwandmenge für einen effizienten Spritzvorgang mittels des mit Batterie elektrisch betriebenen Handsprühgerätes.

Die Erfindung zeigt ferner die technische Ausführung und Anwendung auf, bezüglich der Integration der Elektrolysezelle und der Mikropumpe im Sprühkopf, zur Herstellung der oxidativen Radikale im Sprühwasser zur Reinigung von Oberflächen und zur Elimination von Keimen.

Die Erfindung bildet ein integriertes System, mit den technischen Komponenten für die elektrolytische Herstellung von oxidativen Radikalen im Wasser mittels Volldiamant Elektroden, wobei ferner in der Sprühdüse eine Elektrode zur elektrostatischen Aufladung der Sprühtröpfchen enthalten sein kann.

Dabei liegt der Schwerpunkt der Innovation nicht nur in der technischen Ausführung des Batterie betriebenen Handsprühgerätes mit einer Elektrolysezelle zur Herstellung von oxidativen Radikalen, sondern auch in der neuen Applikations- Technik der kombinierten Anwendung des Handsprühgerätes mit einer wässrigen Lösung aus oxidativen Radikalen, die Dank einer ultraschnellen Superoxidation, nicht nur reinigen sondern auch desinfizieren können und sogar in der Lage sind, Biofilme aufzulösen.

In mehrjährigen Versuchen wurden die Prozesstechniken des Handsprühgerätes optimiert, Konzentrationen von oxidativen Radikalen im Wasser spezifiziert und die spezifischen Parameter den technischen Anforderungen angepasst und auch Behandlungszeiten eruiert, um eine perfekte Reinigung und Desinfektion auf allen Arten von Oberflächen, Materialien und Oberflächen, zu erreichen.

Der Erfinder hat in mehrjähriger Forschung- und Entwicklungsarbeit im Labor und im praktischen Einsatz das technisch neu entwickelte Batterie betriebene Handsprühgerät getestet und perfektioniert und eine biozide Effizienz von 99.9% erreicht. Nach Kenntnisstand des Erfinders ist bis heute kein Batterie betriebenes Handsprühgerät auf dem Gebiet der Desinfektion und Reinigung von Oberflächen, Materialien und Gegenständen vorhanden, das mittels elektrolytisch, mit Volldiamant-Elektroden hergestelltem Wassers und mit den daraus erzeugten oxidativen Radikalen als Biozid gegen Keime, Schimmelpilze, Viren und Bakterien etc. funktioniert.

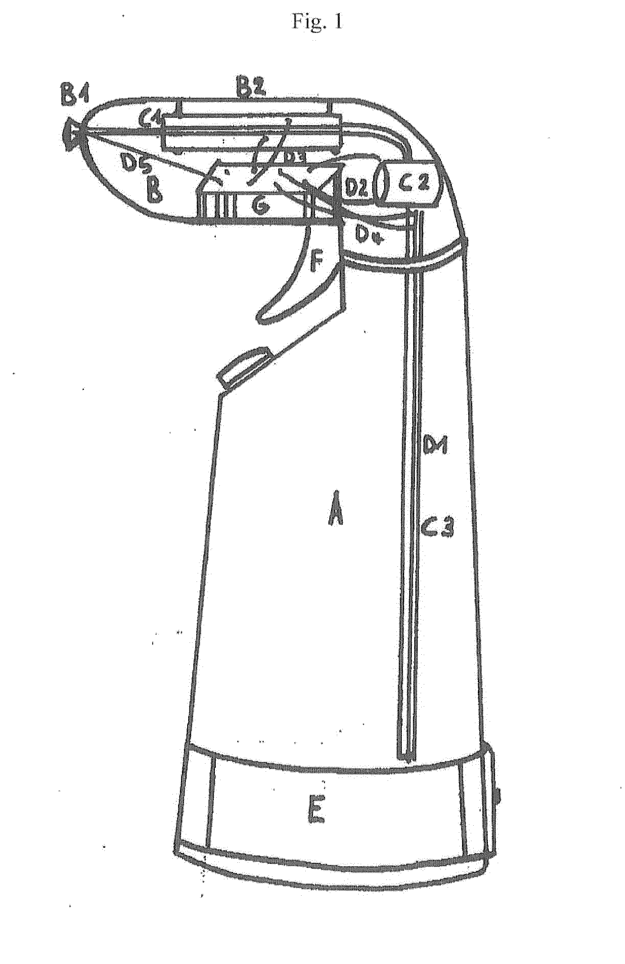

Die Erfindung wird nun anhand der einzigen Figur, Fig. 1, die schematisch ein Ausführungsbeispiel eines Batterie betriebenen mobilen Handsprühgerätes zeigt, näher beschrieben.

Das in Fig. 1 gezeigte Handsprühgerät besteht im Wesentlichen aus zwei Teilen, einem Flüssigkeitsbehälter A, insbesondere aus Kunststoff, mit einem Einfüllstutzen mit einem Verschlussdeckel aus einem nicht oxidierenden Material, und einem Sprühkopf B enthaltend eine Sprühdüse B 1 mit eingebauter Elektrode zur elektrostatischen Aufladung von Wassertröpfchen. Der Sprühkopf B weist ein Sprühgehäuse B2, insbesondere aus Kunststoff auf, welches mit dem Behälter A verschraubbar ist. Das Sprühgehäuse B2 enthält eine Elektrolysezelle C1 aus einem nicht oxidierenden Kunststoff mit Volldiamant-Elektroden, insbesondere mit zumindest zwei Elektroden, einer Anode und einer Kathode, aus mit Bor oder mit anderen Mineralien dotiertem Volldiamant oder mit einer Beschichtung aus dotiertem Diamant und mit elektrischen Anschlussmuffen mit positivem und negativem Pol, welche mit einer elektrischer Polumkehr-Steuereinheit verbunden sind. Im Sprühgehäuse B2 befindet sich eine elektrische Miniatur-Wasserpumpe C2 aus nicht oxidierbarem Material mit elektrischem Anschluss. Schläuche C3 aus Teflon oder einem anderen geeigneten Material dienen als Steigrohr und als Verbindungsleitungen vom Flüssigkeitsbehälter A zur Wasserpumpe C2, von der Wasserpumpe C2 zur Elektrolysezelle C1 und von der Elektrolysezelle C1 zur Sprühdüse B1 und weisen entsprechende Anschlussmuffen auf. Mit D1, D2, D3, D4 und D5 sind elektrische Drahtverbindungen von der Batterie zu den Verbrauchern bezeichnet. Die in der Sprühdüse B1 befindliche Elektrode ist vorzugsweise stäbchenförmig und besteht insbesondere aus Titan. Das Handsprühgerät weist im unteren Teil ein vom Flüssigkeitsbehälter A abgetrenntes Batteriefach E mit einem Deckel auf, welches auswechselbare und wieder aufladbare Batterien enthält, die mit einer integrierten Ladeanzeige mit LED Lämpchen und mit einem Anschlussstecker mit Kabel für das Wiederaufladen der Batterien versehen sind. Die Batterien weisen eine Leistung von optimal etwa 30 W/h auf. Am Sprühkopf B ist ein als Elektroschalter ausgeführter Sprüh-Fingerabzug F zum on- und off- Betrieb vorgesehen. Eine elektrische bzw. elektronische Steuerung G befindet sich auf einer Platinen-Matrix und ist mit elektrischen Drahtverbindungen mit den Stromverbrauchern und dem Fingerabzug F des Handsprühgerätes verbunden.

Die Wasserpumpe C2, die flexiblen Verbindungsleitungen, das Steigrohr im Flüssigkeitsbehälter A und das vom Flüssigkeitsbehälter A getrennte Batteriefach F sind aus oxidationsmittelbeständigem Kunststoff gefertigt.

Die Volldiamant-Elektroden sind insbesondere Diamantpartikel-Elektroden, bei welchen dotierte Volldiamantpartikel in ein elektrisch isoliertes Trägermaterial, beispielsweise in Kunststoff, verankert sind. Derartige Elektroden werden in der Elektrolysezelle vorzugsweise als bipolare Elektroden verwendet.

Die Herstellung der bioziden oxidativen Radikale in wässriger, wahlweise salzhaltiger Lösung erfolgt durch das im Handsprühgerät neu verwendete Elektrolyseverfahren.

Im neuen Verfahren erfolgt die Elektrolyse mittels Volldiamant-Elektroden. Dabei entsteht ein Cocktail aus oxidativen Radikalen nahe am "neutralen Bereich" mit einem pH von 7,4 bis 8,2. An der Anode werden neben OH- Hydroxylgruppen und O₃ vor allem freies Chlor (Cl₂) gebildet, die mit den Hydroxylgruppen zur Bildung von Hypochlorid HOCI und Hypochlorid Säure H₂OCl führen, die organisch sehr rasch abgebaut werden. Um die Elektrolyse des Wassers bezüglich Stromverbrauchs günstiger und besser durchführen zu können, kann dem Wasser wegen der verbesserten Elektrokonduktivität Salz, insbesondere NaCl oder ein anderes Salz, wie CaCl oder KCl, in Form von Pastillen oder Chips oder Kristallpulver, zugemischt werden.

Bei der Elektrolyse dieser Salzverbindungen zusammen mit den natürlich im Trinkwasser vorkommenden Mineralien entstehen zudem oxidierende Moleküle, wie reduzierendes Peroxyd-Di-Sulfat, Peroxyd-Di-Phosphat und Percarbonat.

Die NaCl Salzkonzentration beträgt pro Liter Wasser vorzugsweise 0,5-8 Gramm NaCl (Kochsalz) oder mehr.

Das erfindungsgemäße Handsprühgerät wird wie folgt gehandhabt. Als erster Arbeitsschritt wird das Handsprühgerät mittels Kabel und Ladegerät an das 220 V Stromnetz angeschlossen, um die Batterien voll aufzuladen. Anschliessend wird der Flüssigkeitsbehälter A über den Einfüllstutzen bis zur Voll-Markierung aufgefüllt und der Stutzen mit dem Deckelverschluss fest verschlossen. Mit dem Fingerabzug F am Sprühkopf B wird der elektrische Kreislauf ein- und ausgeschaltet. Beim Einschalten des Handsprüh- Gerätes wird die Mikro-Wasserpumpe C₂ eingeschaltet und die Pumpe C₂ schöpft das Wasser über das Steigrohr zur Elektrolysezelle C₁, die ebenfalls unter Spannung steht. Das Wasser wird durch die Elektrolysezelle C₂ zwischen den Volldiamant-Elektroden Anode und Kathode getrieben und wird durch die elektrolytischen Prozesse chemisch aufgespaltet, was zur Bildung von oxidativen Radikalen führt. Anschließend wird das elektrolysierte Wasser zur Sprühdüse B₁ transportiert. Falls in der Sprühdüse B₁ eine stäbchenförmige Titan-Elektrode eingebaut ist werden die Tröpfchen elektrostatisch aufgeladen.

Der Sprühvorgang kann somit beliebig fortgesetzt oder abgebrochen werden. Zu beachten sind, dass der Wasserstand und die Ladung der Batterie regelmässig kontrolliert werden, damit das Handsprühgerät immer einwandfrei funktioniert. Der Ladezustand der Batterie oder des Akkus wird durch eine grüne oder rote Leuchtdiode immer während der Funktionsdauer angezeigt. Bei rotem Aufleuchten müssen die Batterien oder der Akku neu aufgeladen oder durch vollgeladene Austausch -Elemente ersetzt werden.

Der ideale Sprüh-Abstand zur Oberfläche beträgt ca. 30 cm. Die Oberflächen müssen grosszügig benetzt werden und anschliessend mit einem trockenen Mikro-Fasertuch abgerieben und getrocknet werden. Wo Hochglanz nicht erforderlich ist, ist es auch möglich, das Sprühwasser einfach eintrocknen zu lassen.

Das reduzierende Wasser wirkt wie ein Seifenprodukt und entfernt nicht nur Schmutz und bakterielle Schmutz- und Biofilme, sondern desinfiziert ebenfalls durch die Abtötung von 99,9 % aller Mikroorganismen, wie Viren, Gramm-positive und Gramm-negative Bakterien, Hefen, Protozoen durch Superoxidationen etc. in Sekunden. Das oxidative Wasser hat eine verlängerte Wirkungszeit, was die Desinfektions- Intensität begünstigt. Die Reinigung ist perfekt und es entstehen keine toxischen Rückstände.

Das Reinigungs- Verfahren mit dem Batterie betriebenen Handspraygerät und ElektrolyseWasser mit oxidativen Radikalen ist billiger als jedes andere Reinigungs-Verfahren mit Chemie. Der Energieverbrauch beträgt lediglich rund 30 W/h.

## Patentansprüche

1. Batterie betriebenes Handsprühgerät mit einem Flüssigkeitsbehälter (A), einem Sprühkopf (B) mit Sprühdüse (B₁), einer Elektrolysezelle (C₁), einer Wasserpumpe (C₂), Steigrohr und Leitungen, einer elektrischen/elektronischen Steuerung (G) mit einem Fingerabzug (F) und einem Batteriefach (E),
**dadurch gekennzeichnet,**
**dass** die Elektrolysezelle (C₁) dotierte Volldiamant-Elektroden enthält.

2. Handsprühgerät nach Anspruch 1, **dadurch gekennzeichnet, dass** die Elektrolysezelle (C₁) im Sprühkopf (B) positioniert ist.

3. Handsprühgerät nach Anspruch 1 oder 2, **dadurch gekennzeichnet, dass** die Elektrolysezelle mit Bor dotierte Volldiamant-Elektroden enthält.

4. Handsprühgerät nach einem der Ansprüche 1 bis 3, **dadurch gekennzeichnet, dass** die Volldiamant-Elektroden Volldiamantpartikel-Elektroden sind.

5. Handsprühgerät nach Anspruch 4, **dadurch gekennzeichnet, dass** die Volldiamantpartikel in einem elektrisch isolierenden Trägermaterial verankert sind.

6. Handsprühgerät nach einem der Ansprüche 1 bis 3, **dadurch gekennzeichnet, dass** die Volldiamant-Elektroden mit Diamant beschichtete Elektroden sind.

7. Handsprühgerät nach einem der Ansprüche 1 bis 6, **dadurch gekennzeichnet, dass** die Anode und die Kathode der Elektrolysezelle dotierte Volldiamant-Elektroden sind.

8. Handsprühgerät nach Anspruch 1, **dadurch gekennzeichnet, dass** in die Sprühdüse (B₁) eine Elektrode zur elektrostatischen Aufladung der Wassertröpfchen eingebaut ist.

9. Handsprühgerät nach Anspruch 7, **dadurch gekennzeichnet, dass** die in der Sprühdose (B₁) positionierte Elektrode stäbchenförmig ausgeführt ist und insbesondere aus Titan besteht.

10. Handsprühgerät nach Anspruch 1, **dadurch gekennzeichnet, dass** die Wasserpumpe (C₂) im Sprühkopf (B) eingebaut ist.

11. Handsprühgerät nach Anspruch 1, **dadurch gekennzeichnet, dass** das Batteriefach (E) am Boden des Flüssigkeitsbehälters (A) angeordnet ist.

12. Handsprühgerät nach Anspruch 1, **dadurch gekennzeichnet, dass** sich die elektrische/elektronische Steuerung auf einer Platinen-Matrix befindet, welche im Sprühkopf (B) angeordnet ist.

13. Handsprühgerät nach einem der Ansprüche 1 bis 11, **dadurch gekennzeichnet, dass** es ein Steigrohr und Schlauchverbindungen aus Teflon enthält.

14. Handsprühgerät nach einem der Ansprüche 1 bis 12, **dadurch gekennzeichnet, dass** der Sprühkopf (B) ein Gehäuse (B₂), insbesondere aus Kunststoff, aufweist, welches mit dem Flüssigkeitsbehälter (A), welcher insbesondere ebenfalls aus Kunststoff besteht, verschraubbar ist.

15. Verwendung des Handsprühgerätes nach einem oder mehreren der Ansprüche 1 bis 13, mit einem Wasser, welchem ein Salz, insbesondere NaCl, CaCl oder KCl, insbesondere in einer Menge von 0,5 g bis 8 g pro Liter, zugemischt ist.
